# EUROPEAN PATENT SPECIFICATION

(11) **EP 0 492 093 B1**
(45) Date of publication and mention of the grant of the patent: **17.03.1999**
(21) Application number: 91118720.1
(22) Date of filing: 04.11.1991
(51) Int. Cl.: C07D 207/34, C07D 207/42

(54) **Process for the preparation of 2-aryl 1-substituted-5-(trifluoromethyl)pyrrole compounds useful as insecticidal, acaricidal and nematocidal agents and as intermediates for the manufacture of said agents**
Verfahren zur Herstellung von 2-Aryl-1-substituierten-5-(Trifluormethyl)-Pyrrolen als Insektizide, Akarizide und Nematizide Wirkstoffe sowie als Zwischenprodukte zur Herstellung dieser Wirkstoffe
Procédé pour la préparation de 2-aryl-1-substitué-5-(trifluorométhyl)pyrroles comme agents insecticides, acaricides et nematicides et comme intermediaires pour la production des agents desdits

(30) Priority: 26.12.1990 US 634286
(43) Date of publication of application: 01.07.1992
(73) Proprietor: AMERICAN CYANAMID COMPANY, Wayne, NJ 07470-8426 (US)
(72) Inventor: Doehner, Robert Francis, Jr., East Windsor, New Jersey 08520 (US)
(74) Representative: Wächtershäuser, Günter, Prof. Dr.

(56) References cited:
- EP-A- 0 312 723
- JOURNAL OF ORGANIC CHEMISTRY. vol. 50, no. 20, 1985, EASTON US pages 3816 - 3823; A. PADWA ET AL.: 'Intramolecular Munchnone Cycloadditions: Preparation and Chemistry of the Intramolecular Dipolar Cycloadducts'
- JERRY MARCH 'Advanced Organic Chemistry, second edition' 1983 , MCGRAW-HILL INTERNATINAL BOOK COMPANY , TOKIO
- CHIMIA vol. 29, no. 8, August 1975, AARAU CH pages 350 - 352; R. VERBRUGGEN, G. H. VIEHE: 'Cycloadditions with 2-Chloro-1-Nitroethylene'
- CHEMISCHE BERICHTE vol. 103, no. 8, August 1970, WEINHEIM DE pages 2610 - 2624; R. HUISGEN ET AL.: 'Eine bequeme Synthese von N-substituierten Pyrrolen aus mesoionischen Oxazolonen und Alkinen'

## Description

In view of the fundamental utility of certain arylpyrrole compounds for the control of insect, acarid and nematode pests and for the protection of important agronomic crops from the ravages of said pests, more methods of preparation of said arylpyrrole compounds are needed. It is an object of this invention to provide an efficient and effective single step process for the preparation of insecticidal 2-aryl-1-substituted-5-(trifluoromethyl)pyrrole compounds.

The present invention relates to a process for the preparation of arylpyrrole compounds of formula I 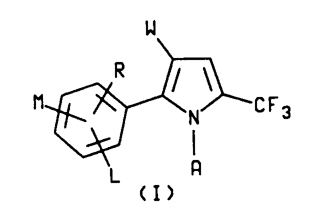 wherein
- A: is C₁-C₆ alkyl, phenyl or C₁-C₆ alkyl substituted with phenyl;
- W: is CN, NO₂ or COOR₁;
- L: is hydrogen or halogen and
- M and R: are each independently hydrogen, C₁-C₄ alkyl, C₁-C₄ alkoxy, C₁-C₄ alkylthio, C₁-C₄ alkylsulfinyl, C₁-C₄ alkylsulfonyl, CN, NO₂, halogen, CF₃, R₂CF₂Z, R₃CO or NR₄R₅ and when M and R are on adjacent positions they may be taken together with the carbon atoms to which they are attached to form a ring in which MR represents the structure -OCH₂O-, -OCF₂O- or -CH=CH-CH=CH-;
- Z: is S(O)ₙ or O;
- R₁: is C₁-C₆ alkyl, C₃-C₆ cycloalkyl or phenyl;
- R₂: is hydrogen, F, CHF₂, CHFCl or CF₃;
- R₃: is C₁-C₄ alkyl, C₁-C₄ alkoxy or NR₃R₄;
- R₄: is hydrogen or C₁-C₄ alkyl;
- R₅: is hydrogen, C₁-C₄ alkyl or R₆CO;
- R₆: is hydrogen or C₁-C₄ alkyl; and
- n: is an integer of 0, 1 or 2 which comprises reacting a 2-arylamino acid compound of formula II 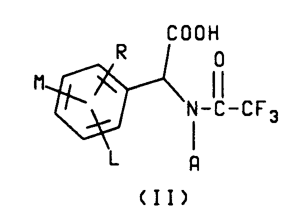 wherein
- A, L, M and R: are as defined above with about one molar equivalent of a 1,3-dipolarophile of formula III 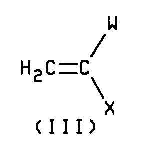 wherein
- X is Cl or Br and W: is defined above and at least one molar equivalent of an organic base in the presence of an acid anhydride and a solvent at an elevated temperature.

The product arylpyrrole compounds of formula I are highly useful as insecticidal, acaricidal and nematocidal agents and, further, are important intermediates in the manufacture of certain insecticidal arylpyrrole compounds. Compounds of formula II and a method for the preparation thereof are described in copending U.S. application Serial Number 06/634,287.

Advantageously, it has been found that a 2-arylamino acid compound of formula II efficiently undergoes a one step 1,3-cycloaddition to form a 5-membered heterocyclic compound of formula I.

Hence, arylpyrrole compounds of formula I may be prepared by reacting a 2-arylamino acid compound of formula II with about one molar equivalent of a 1,3-dipolarophile of formula III and at least one molar equivalent of an organic base in the presence of an acid anhydride and a solvent, preferably at an elevated temperature. The reaction is shown in flow diagram I

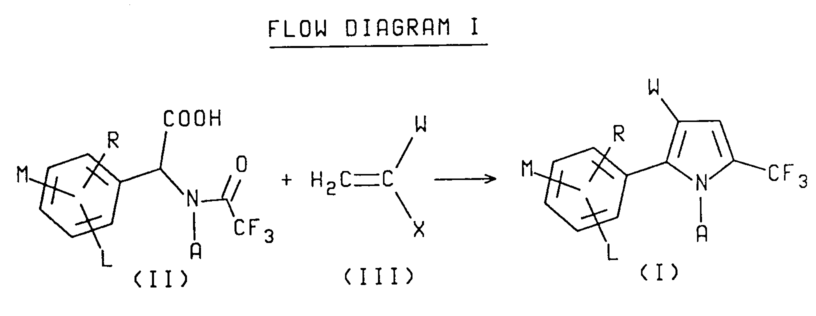

Solvents that may be used in the method of invention include aprotic organic solvents for example nitriles such as acetonitrile; esters such as ethyl acetate and methyl propionate; ethers such as diethyl ether, tetrahydrofuran, dioxane, ethylene glycol dimethyl ether and the like; halogenated hydrocarbons such as methylene chloride, 1,2-dichloroethane, chloroform, 1,1,1-trichloroethane, and carbon tetrachloride; carbonic acid amides such as N,N-dimethylformamide and N-methylpyrrolidinone; sulfoxides such as dimethyl sulfoxide; sulfones such as tetramethylene sulfone; aromatic hydrocarbons such as benzene, toluene, and xylene; halogenated aromatic hydrocarbons such as chlorobenzene and o-dichlorobenzene. One of the preferred organic solvents is acetonitrile. Acid anhydrides suitable for use in the method of invention are lower-alkyl anhydrides such as acetic anhydride. Among the organic bases that may be used in the inventive method are pyridine, morpholine, tri(C₁-C₄)alkylamine, hexamethylenetetramine, and dimethylamino pyridine. A preferred organic base is a tri(C₁-C₄)alkylamine such as triethylamine.

The rate of formation of the formula I arylpyrrole is directly related to the reaction temperature. Lower reaction temperatures result in increased reaction time. Advantageously, it has been found that elevated temperatures such as 30°-100°C, preferably 50°-90°C result in efficient product formation.

Compounds of formula II may be prepared via condensation of the appropriate arylaldehyde with cyanide and a suitable amine to form the corresponding amino nitrile, which is acetylated and hydrolyzed to form the amino acid intermediate of formula III, which is then trifluoroacetylated to obtain the desired formula II compound as shown in Flow Diagram II.

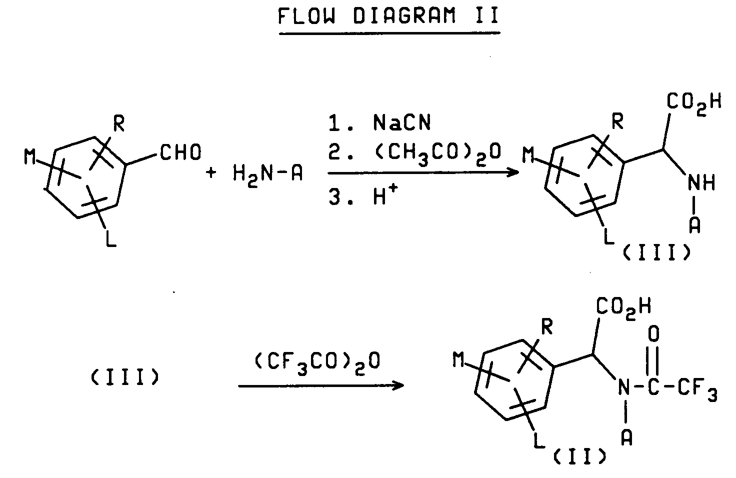

Compounds of formula II may also be prepared via trifluoroacetylation of the appropriate arylglycine precursor followed by alkylation of the thus-obtained compound using an alkylating agent such as a lower alkylhalide. Using methyliodide as the alkylating agent, the reaction sequence is illustrated in flow diagram III.

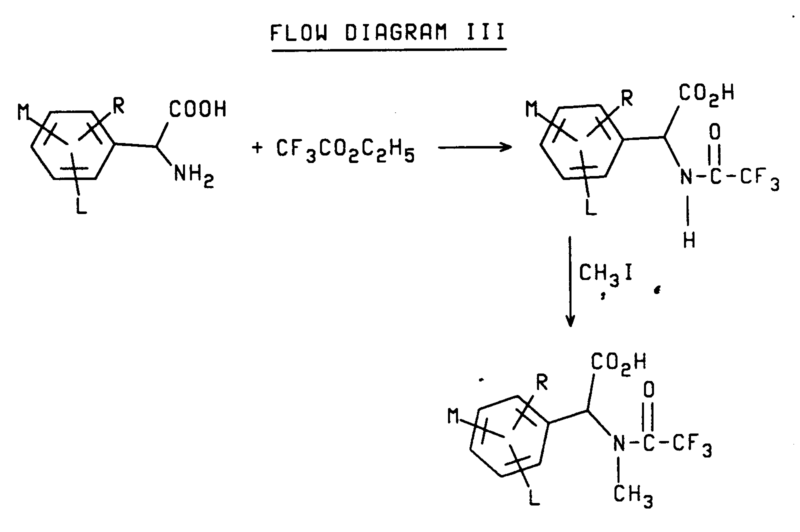

Compounds of formula I are useful for the control of insect, acarid and nematode pests and for protecting growing and harvested crops from the ravages of said pests. Compounds of formula I are also useful as key intermediates in the manufacture of certain insecticidal arylpyrrole compounds. For example, compounds of formula I may be halogenated to afford the corresponding 2-aryl-4-halopyrrole insecticidal agents of formula IV as shown in flow diagram IV.

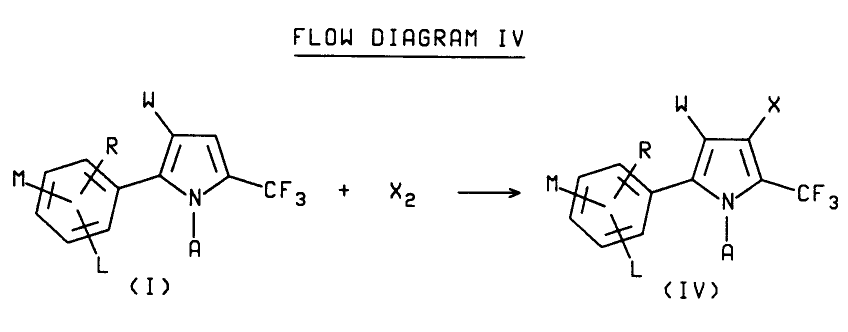

By varying the substituents A, W, L, M and R and the halogen, X₂, numerous possible insecticidal arylpyrroles may be prepared from the key intermediate compound of formula I.

In order to facilitate a further understanding of the invention, the following examples are set forth primarily for the purpose of illustrating certain more specific details thereof. The invention is not to be limited thereby except as defined in the claims. The term HPLC designates high pressure liquid chromatography.

### EXAMPLE 1

### Preparation of 2-(p-Chlorophenyl)-1-methyl-5-(trifluoromethyl)pyrrole-3-carbonitrile

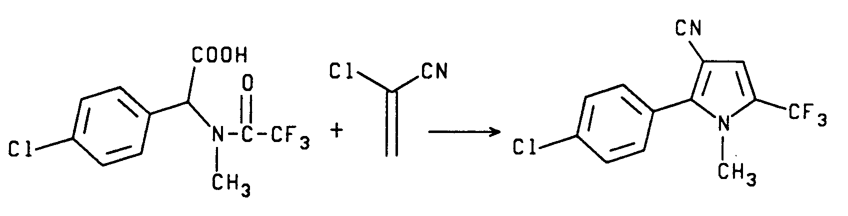

A mixture of 2-(p-chlorophenyl)-N-(trifluoroacetyl)sarcosine (147.7 g, 0.50 mol) in acetonitrile is treated dropwise with α-chloroacrylonitrile (54.7 g, 0.62 mol) and acetic anhydride (153 g, 1.5 mol), stirred vigorously, treated dropwise with triethylamine (58.1 g, 0.57 mol) at 56-60°C over a 1 1/4 hour period, heated at 60°C for 16 hours and concentrated in vacuo to give a residue. The residue is partitioned between ethyl acetate and water. The organic phase is concentrated in vacuo to afford an amber solid residue. Said solid is purified by flash chromatography (silica; ethyl acetate/hexanes) and recrystallized from methanol to give the title product as a pale yellow solid, mp 129.5-130.5°C.

### EXAMPLE 2

### Preparation of 2-Aryl-1-(substituted)-5-(trifluoromethyl)pyrrole compounds

Using essentially the same procedure described in Example 1 and substituting the appropriate 2-aryl-N-(trifluoroacetyl)substrate and suitable 1,3-dipolarophile yields the title products shown in Table I.

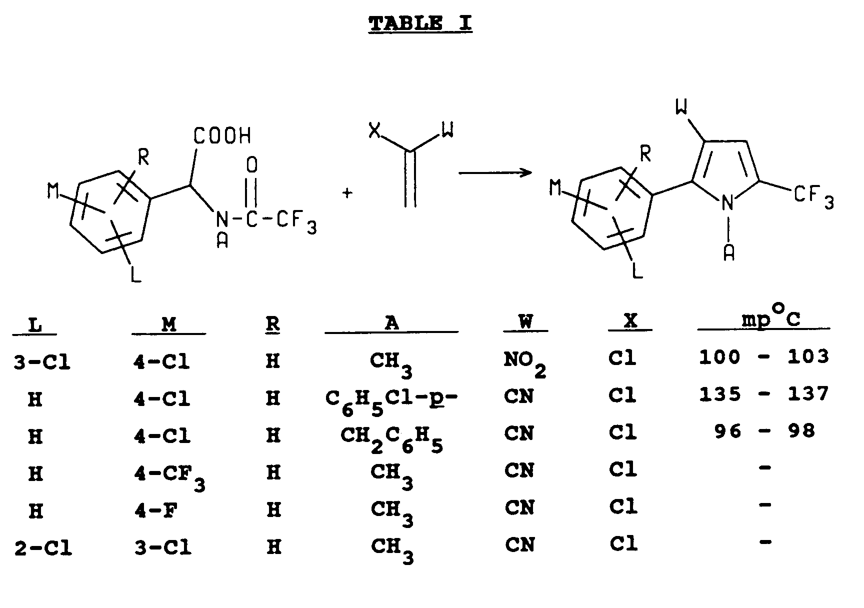

### EXAMPLE 3

### Preparation of 2-(p-Chlorophenyl-4-bromo-1-methyl-5-(trifluoromethyl)pyrrole-3-carbonitrile

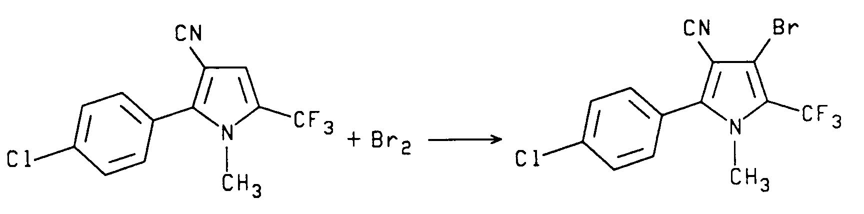

A solution of 2-(p-chlorophenyl)-1-methyl-5-(trifluoromethyl)pyrrole-3-carbonitrile (5.70 g, 0.02 mol) in chlorobenzene is treated with bromine (3.52 g, 0.022 mol), heated at 80°C for 20 hours, cooled to room temperature, treated with additional bromine (3.52 g, 0.022 mol) and heated at 100°C until reaction is complete by HPLC analysis. The reaction mixture is cooled to room temperature and diluted with ethyl acetate and water. The organic phase is washed with aqueous sodium metabisulfite, dried (MgSO₄) and concentrated in vacuo to afford a solid residue. The residue is recrystallized from ethyl acetate/heptane to give the title product as a white solid, 6.50 g (89.4% yield), mp 126-129°C.

## Claims

1. A process for the preparation of a compound characterized by formula I 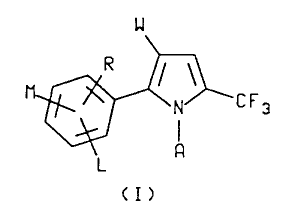 wherein
A is C₁-C₆ alkyl, phenyl or C₁-C₆ alkyl substituted with phenyl;
W is CN, NO₂ or COOR₁;
L is hydrogen or halogen and
M and R are each independently hydrogen, C₁-C₄ alkyl, C₁-C₄ alkoxy, C₁-C₄ alkylthio, C₁-C₄ alkylsulfinyl, C₁-C₄ alkylsulfonyl, CN, NO₂, halogen, CF₃, R₂CF₂Z, R₃CO or NR₄R₅ and when M and R are on adjacent positions they may be taken together with the carbon atoms to which they are attached to form a ring in which MR represents the structure -OCH₂O-, -OCF₂O- or -CH=CH-CH=CH-;
Z is S(O)ₙ or O;
R₁ is C₁-C₆ alkyl, C₃-C₆ cycloalkyl or phenyl;
R₂ is hydrogen, F, CHF₂, CHFCl or CF₃;
R₃ is C₁-C₄ alkyl, C₁-C₄ alkoxy or NR₄R₅;
R₄ is hydrogen or C₁-C₄ alkyl;
R₅ is hydrogen, C₁-C₄ alkyl or R₆CO;
R₆ is hydrogen or C₁-C₄ alkyl; and
n is an integer of 0, 1 or 2 which comprises reacting a compound of formula II 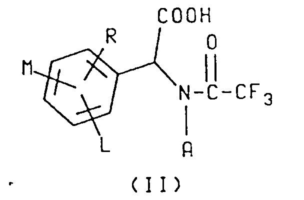 wherein
A, L, M and R are as defined above with one molar equivalent of a compound of formula III 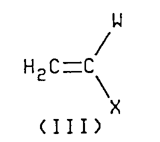 wherein
X is Cl or Br and W is described above and at least one molar equivalent of an organic base in the presence of an acid anhydride and a solvent at a temperature of 30°C to 100°C.

2. The process according to claim 1 wherein the organic base is tri(C₁-C₄ alkyl)amine and the solvent is acetonitrile.

3. The process according to claim 2 wherein the organic base is triethylamine.

4. The process according to claim 1 wherein X is Cl and W is CN or NO₂.

## Patentansprüche

1. Verfahren zur Herstellung einer Verbindung, gekennzeichnet durch Formel I 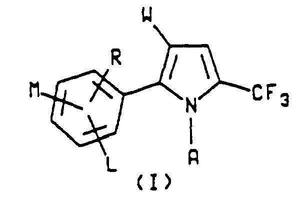 worin
A C₁-C₆-Alkyl, Phenyl oder C₁-C₆-Alkyl, substituiert mit Phenyl, darstellt;
W CN, NO₂ oder COOR₁ darstellt;
L Wasserstoff oder Halogen darstellt und
M und R jeweils unabhängig Wasserstoff, C₁-C₄-Alkyl, C₁-C₄-Alkoxy, C₁-C₄-Alkylthio, C₁-C₄-Alkylsulfinyl, C₁-C₄-Alkylsulfonyl, CN, NO₂, Halogen, CF₃, R₂CF₂Z, R₃CO oder NR₄R₅ darstellen, und, wenn M und R an benachbarten Stellungen vorliegen, sie mit den Kohlenstoffatomen, an die sie gebunden sind, unter Bildung eines Rings, in dem MR die Struktur -OCH₂O-, -OCF₂O- oder -CH=CH-CH=CH-wiedergibt, zusammengenommen werden können;
Z S(O)ₙ oder O darstellt;
R₁ C₁-C₆-Alkyl, C₃-C₆-Cycloalkyl oder Phenyl darstellt;
R₂ Wasserstoff, F, CHF₂, CHFCl oder CF₃ darstellt;
R₃ C₁-C₄-Alkyl, C₁-C₄-Alkoxy oder NR₄R₅ darstellt;
R₄ Wasserstoff oder C₁-C₄-Alkyl darstellt;
R₅ Wasserstoff, C₁-C₄-Alkyl oder R₆CO darstellt;
R₆ Wasserstoff oder C₁-C₄-Alkyl darstellt und
n eine ganze Zahl von 0, 1 oder 2 ist,
das Umsetzen einer Verbindung der Formel II 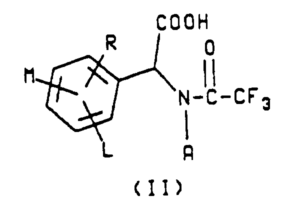 worin
A, L, M und R wie vorstehend definiert sind, mit einem Moläquivalent einer Verbindung der Formel III 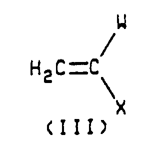 worin
X Cl oder Br darstellt und W wie vorstehend beschrieben ist und mindestens einem Moläquivalent einer organischen Base in Anwesenheit eines Säureanhydrids und eines Lösungsmittels bei einer Temperatur von 30°C bis 100°C umfaßt.

2. Verfahren nach Anspruch 1, wobei die organische Base Tri(C₁-C₄-alkyl)amin darstellt und das Lösungsmittel Acetonitril darstellt.

3. Verfahren nach Anspruch 2, wobei die organische Base Triethylamin darstellt.

4. Verfahren nach Anspruch 1, wobei X Cl darstellt und W CN oder NO₂ darstellt.

## Revendications

1. Procédé pour la préparation d'un composé caractérisé par la formule I 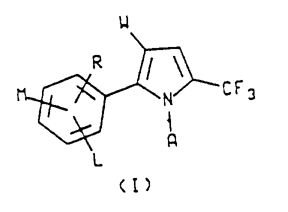 dans laquelle
A est un groupe alkyle en C₁-C₆, phényle ou alkyle en C₁-C₆ substitué par un groupe phényle;
W est CN, NO₂ ou COOR₁;
L est un atome d'hydrogène ou d'halogène; et
M et R sont chacun indépendamment un atome d'hydrogène, un groupe alkyle en C₁-C₄, alcoxy en C₁-C₄, alkylthio en C₁-C₄, alkylsulfinyle en C₁-C₄, alkylsulfonyle en C₁-C₄, CN, NO₂, un atome d'halogène, CF₃, R₂CF₂Z, R₃CO ou NR₄R₅ et, lorsque M et R sont sur des positions adjacentes, ils peuvent être pris ensemble avec les atomes de carbone auxquels ils sont fixés pour former un cycle dans lequel MR représente la structure -O-CH₂-O-, -OCF₂O- ou -CH=CH-CH=CH-;
z est S(O)ₙ ou O;
R₁ est un groupe alkyle en C₁-C₆, cycloalkyle en C₃-C₆ ou phényle;
R₂ est un atome d'hydrogène, F, CHF₂, CHFCl ou CF₃;
R₃ est un groupe alkyle en C₁-C₄, alcoxy en C₁-C₄ ou NR₄R₅;
R₄ est un atome d'hydrogène ou un groupe alkyle en C₁-C₄;
R₅ est un atome d'hydrogène, un groupe alkyle en C₁-C₄ ou R₆CO;
R₆ est un atome d'hydrogène ou un groupe alkyle en C₁-C₄; et
n est un nombre entier qui est soit 0, 1 ou 2,
qui comprend la réaction d'un composé de formule II 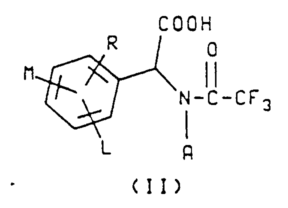 dans laquelle
A, L, M et R sont tels que définis ci-dessus, avec un équivalent molaire d'un composé de formule III 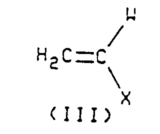 dans laquelle
X est Cl ou Br, et W est tel que décrit ci-dessus, et au moins un équivalent molaire d'une base organique en présence d'un anhydride d'acide et d'un solvant à une température de 30°C à 100°C.

2. Procédé selon la revendication 1, dans lequel la base organique est une tri(alkyl en C₁-C₄)amine et le solvant est l'acétonitrile.

3. Procédé selon la revendication 2, dans lequel la base organique est la triéthylamine.

4. Procédé selon la revendication 1, dans lequel X est Cl et W est CN ou NO₂.
